# EUROPEAN PATENT APPLICATION

(11) **EP 3 159 445 A1**
(43) Date of publication of application: **26.04.2017**
(21) Application number: 15810501.5
(22) Date of filing: 18.06.2015
(51) Int. Cl.: D04H 1/541

(54) **NON-WOVEN FABRIC**

(30) Priority: 18.06.2014 ES 201430930
(71) Applicant: BC Nonwovens, S.L., 08036 Barcelona (ES)
(72) Inventor: VIÑAS PICH, Carlos, 08036 Barcelona (ES)
(74) Representative: Isern-Jara, Nuria
(86) International application number: PCT/ES2015/070480
(87) International publication number: WO 2015/193530

(57) **Abstract**

The present invention relates to a non-woven fabric for a non-woven air filtering substrate and an ADL substrate of fibre-based three-dimensional multi-layer structure, comprising at least 4 layers arranged one above another, arranged securely, wherein there is a first, upper layer in contact with the flow to be treated, which comprises fibres of a fineness ranging from 6 to 20 dtex and lengths ranging from 32 to 80 mm, and wherein the remaining layers comprise fibres of a fineness ranging from 0.6 to 10 dtex and lengths ranging from 12 to 64 mm. The present invention also relates to a method for producing the non-woven fabric that is the subject matter of the invention and also to the use thereof for preparing sanitary products or filters.

## Description

### OBJECT OF THE INVENTION

The present invention falls within the field of materials, specifically the field of non-woven fabrics, in particular those used for the manufacture of air filters or sanitary products with a multi-layer structure of a high quality and high liquid filtration or acquisition and distribution efficiency.

The present invention relates to a non-woven fabric and a filter with said non-woven fabric that has multiple technical advantages such as preventing the common rapid clogging of the filtering surface due to large particles (hampering the passage of the flow through the filter), thus lengthening the useful life of the product. In addition, the present invention also relates to a sanitary product with an acquisition and distribution of liquid substrate.

The present invention also relates to a method for manufacturing a non-woven material, in particular a three-dimensional structure used as an air purification filter or as a sanitary product with at least four layers arranged one above another made of different materials.

The present invention also relates to the use of a three-dimensional material, object of the present invention, as an air purifying filter. In particular, the use of the filter of the present invention is used in any industrial device or process where high-efficiency air purification is necessary, such as for example, in the paper, chemical, textile, pharmaceutical or automotive industry, etc.

### BACKGROUND OF THE INVENTION

The non-woven materials, or non-woven fabrics (hereinafter NWF) are a type of textile material made when a network of fibres, which are joined together by mechanical, thermal or chemical methods, but without being woven and without it being necessary to convert the fibres into thread (warp), is produced. The textile non-woven material is therefore a sheet, veil or web of flexible and porous fibres, without a defined weft or interweaving; whereas normal fabric which has a structure produced by the interweaving of a set of threads (warp) with another set of threads (weft) forming angles close to 90°.

The non-woven materials are technological products that may have a limited life, a single use or long life. Some characteristics of non-woven materials are a high level of impermeability, high elasticity, softness, fire resistant, washable, sterilisable, they provide a good barrier against bacteria, etc. These properties mean that NWFs are very versatile products that can be used in a number of industries such as the automotive, pharmaceutical, chemical, medical, geotextile industries, etc.

There are several technologies to manufacture a non-woven material. Generally, the paper, textile and plastic industry have a lot of influence on the current technologies today. In a practical sense, non-woven materials may basically be classified according to their manufacturing process, raw materials, characteristics of the fibres and filaments, bonding process, grammage, transformation or conversion process, or the association of all these elements.

In short, in relation to the methods for producing NWF, although several different technologies are known for the preparation of these NWFs, the most relevant differences are focused, on the one hand, on the type of raw material used and the feeding/processing thereof in the production line and, on the other hand, in the way said raw material is bonded/entangled in order to form the final fabric (NWF).

In order to understand the general process for producing a non-woven fabric, it must be known that the general stages known in the state of the art for making a NWF are the following:
a) Web forming. The web, a structure that is still not bonded, is formed by one or more layers of fibre or filament veils obtained through three different processes:
   a.1) Dry laid: This process uses raw materials in the form of fibres. Non-woven materials produced by carding may be included in the dry laid process and Air laid. In the Carding process, the fibres are arranged parallel by cylinders covered by "combing teeth" that form anisotropic webs, these webs being capable of being crossed over in layers. In this air laid process, the fibres are suspended in an air flow and are then collected in a fabric forming the web.
   a.2) Wet laid: This process uses raw materials in the form of fibres. In the wet laid process, the fibres are suspended in an aqueous medium and are then collected through filters by a bed, in the form of a web.
   a. 3) Molten laid: In the molten laid process, non-woven materials made by extrusion are included, which are Spun web or Spun bonded and which are melt blown. These processes use raw materials in the form of polymers (plastics). In the Spun web / Spun bonded process, a thermoplastic polymer is melted through a "head block", then cooled and stretched, and subsequently deposited on a substrate in the form of a veil or web. In the melt blown process, a plastic polymer is melted through an extruder and passing through a "head" with very small holes, a hot air flow immediately solidifies the mass quickly forming very fine fibres, which are blown at very high speeds on a collecting fabric, thus forming the web.
b) Web bonding. After the formation of the veil or the web, bonding must be carried out (joining of the fibres or filaments), which in the majority of non-woven materials also gives a surface finish necessary for the final product. There are three basic methods for the bonding or finishing of the non-woven materials, which in turn may be combined together:
   b. 1) Mechanical (friction):
      b.1.1) Mechanical - needle punched. The fibres or filaments are entangled through the alternate penetration of many needles that have small protruding hooks.
      b.1.2) Mechanical - Spun laced or Hydroentangled. The entangling of the needles is carried out by high-pressure jets of water penetrating the web.
      b.1.3) Mechanical - Stitch bonded. Bonding or finishing process through the insertion of web sewing threads or threadless process, which uses the fibres themselves of the non-woven material to create the seam.
   b.2) Chemical (attached) - Resin bonded. The chemical binders (resins) join the fibres or filaments of the non-woven material. There are several types of resin bonding processes: Resin bonding process through saturation bonding, resin bonding through spray bonding and through foam bonding.
   b.3) Thermobonded. The joins between the fibres or filaments of the non-woven material are carried out by the effect of the heat through the melting of the fibres or filaments themselves. Two methods are used: calender bonding process and through-air bonding process.

There are other particular manufacturing / forming processes, however those mentioned above already represent a large number of non-woven materials. Table 1 summarises the most common methods for preparing known NWFs in the state of the art:

**Table 1: Schematic representation of the most common processes for the preparation of NWF.**

| RAW MATERIAL | FEEDING MODE /LINE PRODUCTION | MODE OF ENTANGLEMENT BONDING |
|---|---|---|
| Continuous filament | Spun laid | Thermal bonding/through air bonding³ Hydroentangling or Spunlace¹. |
| Staple fibres | Carded | Hydroentangling or Spunlace¹. Needle punching² Thermal bonding³ Chemical bonding⁴ |
| Pulp | Wet laid Air laid | Hydroentangling or Spunlace¹. Thermal bonding³ Chemical bonding⁴ |
| ¹A technique by which the fibres are mechanically bonded with pressurised water. | | |
| ²A technique by which the fibres are mechanically entangled with a series of needles | | |
| ³A technique by which the fibres are mechanically entangled by heat treatment with through-air bonding | | |
| ⁴A technique by which the fibres are entangled by the addition of binders, polymers, etc. | | |

As has been stated above, NWFs are very versatile materials that are used in a number of applications. One of the main applications is the use thereof for the preparation of sanitary or cleaning products, such as:
- Skin care wipes,
- Cleaning cloths (home, industrial),
- Medical gauze and plasters,
- Others: Geotextiles, etc.

Another application of NWFs is air filtering or sanitary products and this is the focus of the inventors of the present invention.

In general, regarding air filtering, the premise is simple: the smaller the size of the particle to be retained, it is considered that the efficiency of the filtering substrate is greater. Currently there are high-efficiency filters formed by one or more layers of NWF, which are designed to retain a very fine particle size. The current solution is to develop very dense NWFs populated with fibres that retain these fine particles.

In relation to these aforementioned premises in the state of the art, there are different patents that describe and protect several methods for manufacturing high-quality filtering material, but which are different to that developed by the researchers of the present invention.

In this way, we find document JP2008208475 which discloses a filter for air purification with a three-dimensional structure, which is manufactured continuously, consisting of at least one upper layer, one lower layer, and both being joined by a network of intermediate, monofilament fibres of 100 to 2000 dtex, and adhesive not appearing as a joining method therebetween.

We also find in the state of the art document EP1464381, which discloses an air filter manufactured continuously, which can filter up to 0.3 µm and formed by a porous asymmetric polyfluoroethylene membrane of 5 to 100 µm, on which a layer of fibrous foam material with PE (polyethylene), PP (polypropylene), PET (polyester), PA (polyamide) or fibreglass fibres is applied. The contact angle of the water with the surface is 120 to 140º.

Document WO0020095 discloses a multi-layer air filter manufactured continuously, which has a central filtering layer formed by a dry forming technique, with wood fibres and/or plastic fibres, being subsequently joined by means of heat, one or several layers of plastic fibres of wood pulp or mixture of both, with strengthening mechanical properties.

Document US4687579 discloses the manufacture of a high-capacity air filter (99.99% efficiency) for particles up to 0.3 microns in diameter, operating at temperatures up to 550ºC, manufactured continuously based on a conventional process using Fourdrinier, Rotoformer or similar machines. The filter is made up of 40% quartz fibre and 60% stainless steel fibres.

Moreover, document JP2007023394 discloses a paper filter of up to four layers for volatile particles of up to 50 µm.

Document KR2006010841 discloses an in-line manufacturing process of a high-efficiency, multi-layer air filter, using non-woven material of different characteristics, having, among others, metal fibres or carbon fibres in order to prevent electrostatic energy from being generated in the cotton fibre.

We also find document US2004154769, which discloses a cellulosic and polymeric filter, carried out continuously, which has at least two fibre layers arranged differently, and deposited from the aqueous slurries.

Based on the state of the art found, it is noted that the air filters made in-line, from layers made up of different fibres, and being joined by something other than an adhesive (such as heat for example), are found described in the state of the art.

However, all these filters of the state of the art have the common characteristic that they are designed with very dense NWFs that are populated with fibres to retain very fine particles. Therefore, these products have a major drawback for the final user since the useful life of the filter decreases because with a smaller pore size there is a higher rate of clogging and, therefore, blocking of the filtering surface due to large particles, which prevent the passage of the smaller particles, saturating the filter surface.

As such, it is noted that with the filter, object of the present invention, relevant technical advantages are obtained which could never be achieved with substrates made using other technologies which incorporate thermal or chemical "binders" cited in the patents mentioned above, which are widely quantifiable by means of technical parameters known by those skilled in the art, such as the Dust Holding Capacity (hereinafter DHC), which measures the maximum amount of dust that a filter can retain before a certain load loss or GSM (grammage, measured in g/m²).

As such, taking this limitation of the state of the art into account, an efficient process for producing high-quality, efficient, NWF-based filtering material, which resolves the problems of the current state of the art is needed.

With regards to sanitary products, such as nappies and other incontinence products, sanitary towels, etc., fast uptake and diffusion of the bodily fluid, such as urine, as well as the retention thereof in the core of the nappy is necessary. To do so, a layer responsible for the acquisition and distribution of liquids (ADL) located inside the structure of the nappy is usually used. In the state of the art, ADL with several layers, which are joined by "thermal" and/or "resin bonding" techniques, are used. Currently, manufacturers only have alternatives based on the thermal bonding, chemical bonding or spun bonding techniques, which means that the final quality perceived by users is limited.

The following documents are known:
Document WO2007077214 (A1) describes a sanitary product with a laminated structure in which it uses a liquid acquisition layer made of spunlace technology and another liquid distribution layer made with through-air bonding technology. This laminated configuration entails the use of a method or element to join both layers, such as heat for example, which entails greater complexity and, therefore, manufacturing costs. This configuration also prevents a gradient effect between the upper layers, which are less dense, and the lower layers, which are denser.

Document US6022818 discloses a composite of different entangled NWF that carries out the function of upper ADL (acquisition and distribution of liquid) sheet and absorbent core all in one, incorporating pulp in a large portion of the composition, which has a negative effect on the results of the rewet tests. Moreover, the central portion of the composite which acts as an ADL, does not specify any range of dimensions, which have an important role in achieving the effects of the present invention, or specific fibre materials that are also advantageous.

Document US2003028985 does not refer to nappies or the like and consists of a laminate of different layers, some absorbent but basically "air-laid" technology.

Document EP0557678 focuses on the use of "Spunbond" - hydroentangled (continuous filament), which is different to that used in the present invention.

And document US6107539 discloses a composite of different NWFs that carry out the function of lower sheet, upper sheet and ADL, but it does not describe any technology for the ADL.

Similarly to the case of the air filtering, the use of the present non-woven fabric, object of the present invention, has relevant technical advantages that could never be achieved with non-woven fabrics made by means of other technologies that incorporate thermal or chemical "binders" cited in the aforementioned documents, advantages which are widely quantifiable by means of technical parameters known by those skilled in the art, such as the rewet test that measures the degree of wetting of the fabric on a surface saturated with liquid in accordance with the standard ISO 9073-14:2007 and according to the standard EDANA ERT 151.0-93, the STT (strike-through time) in accordance with the standard ISO 9073-13:2006 and standard EDANA ERT 150.2-93, which measures the time it takes for a known volume of liquid to pass through a non-woven material which is in contact with an underlying, dry absorbent pad, or the GSM (grammage, measured in g/m²).

In conclusion, the limitation of the aforementioned state of the art means that it is necessary to develop an efficient process for producing a non-woven fabric that may be used for air filtering or as a high-quality and efficient ADL substrate, which resolves the problems of the current state of the art.

### BRIEF DESCRIPTION OF THE FIGURES

As a complement to the description provided herein, and for the purpose of helping to make the characteristics of the invention more readily understandable, in accordance with a preferred practical embodiment thereof, said description is accompanied by the following figures constituting an integral part of the same, which by way of illustration and not limitation represent the following:
Figure 1 shows a graph that represents the comparative thickness and DHC result in a fibre bonding method by means of thermal/chemical bonding in comparison with the method, object of the present invention (Spunlace). In the present figure, the sample coded as n² 9.2 relates to a composition obtained through the thermal bonding method and the samples coded as n² 2-3-4-8 relate to the same composition for the NWF obtained through the method object of the present invention.
Figure 2 shows a graph that represents the relationship between DHC and GSM in order to obtain a filtering efficiency greater than 99%.
Figure 3 shows a graph that represents the relationship between DHC and the thickness in order to obtain a filtering efficiency greater than 99%.
Figure 4 shows a graph that represents the relationship between DHC and the filtering efficiency.
Figure 5 shows a graph that represents the relationship between DHC, GSM, thickness and the filtering efficiency.
Figure 6 shows a schematic exploded view of a structure of an ADL substrate of the present invention, which may be applied to a sanitary product, such as a nappy.

### DETAILED DESCRIPTION OF THE INVENTION

For the object of the present invention, "non-woven material"; "non-woven structure"; "non-woven fabric"; "NWF" or "non-woven" shall be interchangeably understood as a base textile material made by forming a network with fibres joined by mechanical, thermal or chemical methods, but without being woven and without there being the need to convert the fibres into a thread (warp).

For the object of the present invention, "filtering material" or "filtering substrate" are interchangeably understood as a material comprising a non-woven fabric produced with the method object of the present invention, due to an arrangement in the form of secure superimposed layers, formed by natural or synthetic fibres (of the Viscose, PET, PP type or the like) wherein the first layer (which is in contact with the air flow to be filtered) and the second layer, containing fibres with a grain size greater than the following layers, also formed by natural or synthetic fibres (of the Viscose, PET, PP type or the like).

Furthermore, in the present description, acquisition and distribution of liquids (ADL) substrate" shall be understood as a substrate for acquiring liquids through the upper layers closest to the skin of the user and distributing liquids through the lower layers.

In the present invention, "sanitary product" is understood to comprise any type of product for absorbing bodily fluids, such as nappies, urinary incontinence articles, feminine hygiene articles, etc.

Taking into account the numerous applications and arrangements that the NWF may adopt, the researchers of the present invention have developed a technique for the manufacture of the NWF, object of the present invention. This technique combines the carding and hydroentanglement method for staple fibres (mixture of natural and/or synthetic fibres) and it is that which provides greater technical advantages to the product, for example in terms of:
- Sponginess, softness and thickness. By means of the carding process and then the hydroentanglement process, the spongy and soft feel of the original fibre is maintained. In the case of a sanitary product, it is very important to have the greatest thickness and sponginess possible.
- Resistance against handling. In order for the substrate to not break during the post-processing thereof or the use thereof in the final application (the NWF from this method enables certain elongation). Again in the case of a sanitary product, optimum bursting strength is achieved when the ADL substrate is subjected to longitudinal and transversal tension. This characteristic is of great importance in a nappy or the like.
- Low cost. It enables work to be carried out faster than with the needle punching technique and there is no need to add chemical products or apply long drying times to entangle the fibres.

Due to the combination of the carding process and the hydroentanglement process, as well as the selection of fibres used and the order thereof, an optimal NWF is obtained that serves as a base substrate for the manufacture of high-quality air filters and sanitary ADL products.

In all the blind product tests that have been confidentially carried out with nappies, the NWFs based on the spunlace technique, as described in the present invention, always obtain the best rating. This is largely due to the great smoothness to touch and the elongation capacity thereof. Moreover, in the techniques commonly used in sector, i.e. thermal bonding, chemical bonding or spunbond, the final quality perceived by the users is limited and comparatively lower.

In the case of the filtering substrate developed, this high quality is obtained due to the fact that it has the technical advantages that the filtering substrate developed has an efficiency higher than 99% with a DHC higher than 15 g/200 cm². Moreover, the GSM is comprised between 60 and 120 g/m² and the thickness is comprised between 1 and 3 mm.

With regards to the ADL substrate developed, it also uses the non-woven fabric of the invention that has been made using only and exclusively "carded" and "spunlace" technology. The high quality obtained is due to the technical advantages that the ADL substrate has a GSM comprised between 30 and 90 g/m²; a thickness between 0.6 mm and 3 mm, and more preferably between 1.3 mm and 2.5 mm.

In turn, a sanitary product comprising the acquisition and distribution of liquid substrate described above, and may further comprise a cover and an absorbent core, wherein the ADL substrate may be advantageously arranged between the cover and the absorbent layer.

In relation to the selection of the layers, the materials selected and the arrangement thereof is an essential element in the non-woven fabric of the present invention, as such there is:
a) First and second layers: In the case of the filtering substrate, the two first layers are those intended to retain the largest particles and are completely formed by natural or synthetic fibres (of the Viscose, PET, PP type or the like) of a fineness ranging from 6 to 20 dtex and lengths ranging from 32 to 80 mm. In our process, fibres in this fineness range with lengths smaller than 32 mm do not hydrobond well when passing through the pressurised water process (also known as "jet"). This aspect was determined by putting several examples into practice, which are described below.
   In the case of the ADL substrate, the first two upper layers (which are those that have the thickest fibres) are intended to be in contact with a bodily fluid to be captured, and therefore to capture and quickly direct the urine towards the lower layers, additionally comprising at least one hydrophilic substance that may be, for example, based on biodegradable surfactants, for example fatty acids, esters, etc., or the combination thereof to enhance the urine absorption and direction capacity from the contact area with the user's skin towards the absorbent core. These two first upper layers do not have viscose. The rest of the characteristics do not vary.
b) The third and subsequent layers: In the case of the filtering substrate, these are layers intended to retain the smallest particles and are also formed by natural or synthetic fibres (of the Viscose, PET, PP type or the like) of a fineness ranging from 0.6 to 10 dtex, preferably from 0.6 to 7 dtex, and lengths ranging from 12 to 64 mm. In our process, fibres with a fineness lower than 0.6 dtex or with lengths smaller than 12 mm or greater than 64 mm cannot be processed with assurances when carded. We have also seen that at finenesses greater than 10 dtex in the secondary layers, the space of the weft created between fibres is excessive and the efficiency and DHC fall.

For the ADL substrate, the third and subsequent layers (those that have the finest fibres), are responsible for the final distribution of the urine to the area of the absorbent core of the nappy (which comprises, for example, pulp and superabsorbent polymers SAP) and for forming a first barrier against possible "rewet" once the pulp and SAP of the absorbent core absorbs the urine, achieving a greater and faster dry sensation on the skin than the documents of the state of the art. These lower layers do not have viscose and have fibres of a fineness ranging from 0.6 to 7 dtex.

Furthermore, in the ADL substrate, these lower layers may comprise hydrophobic substances that may be, for example, based on biodegradable surfactants, for example fatty acids, esters, etc., or the combination thereof to enhance the retention of the liquid in the absorbent core. It has been seen that at finenesses greater than 7 dtex in secondary layers, the space of the weft created between fibres is excessive, the "anti-rewet" barrier effect falling in the ADL substrate. However, if the properties of the material chosen to manufacture the fibres has sufficient hydrophobia, it is not necessary to add the hydrophobic substance.

The researchers of the present invention have developed a NWF, for high-efficiency filtering, which in turn prevents the rapid common clogging of the filtering surface carried out by the largest particles (hampering the passage of the flow), and therefore, lengthening the useful life of the product. The ADL substrate developed enables a much faster absorption and distribution of the liquid without obstructing the holes or passages through the fibres.

The filtering substrate and the ADL substrate developed in the present invention are based on a multi-layer NWF also object of the invention, and the novelty and inventive step lie in the use of fibres with different sizes and characteristics in each one of the layers in order to achieve a funnel-type "gradient" without the use of any system, method or element to join them, since they are made securely.

In the case of the filtering substrate, the largest particles are retained in a first level (first and second layers), with the technical advantage that the filtering surface is not clogged and still leaving a passage for the medium and smaller sized particles, which are in turn gradually retained in different levels (subsequent lower layers), but enabling the passage of air flow with microparticles not affected by the filtering, thus ensuring an efficiency of at least 99% and a DHC higher than 15 g/200 cm².

With regards to the ADL substrate, it also benefits from this funnel structure with a "gradient" effect of the NWF, in order to capture and distribute the urine inside a sanitary product. The NWF developed has only and exclusively been carried out by means of carding and hydroentangled technology, and at the end of the process a hydrophilic treatment is added to at least the first layers closest to the skin of the user to improve the urine absorption capacity.

The manufacturing and bonding process of the different fibres to form the different layers is carried out through a carding + hydroentanglement process.

As mentioned above, the NWFs prepared by means of the carding and hyrdoentanglement technology of staple fibres provide certain advantages with respect to other techniques:
- Sponginess, thickness and softness: by means of the carding + hydroentanglement process, the spongy and soft feel of the original fibre is maintained.
- Non-brittle product: it offers a resistance to handling, which is necessary so that the substrate does not break during the use thereof (NWF from this process enables a certain elongation).
- Low cost (it enables work to be carried out faster than with needle punching and there is no need to add chemical products or apply long drying times).

It is especially relevant that the NWF is manufactured by means of this technology, since it provides extra sponginess and thickness that facilitates the gradual retention of particles that has been explained, but in turn ensures certain freedom of passage of the remaining air flow. Furthermore, there is an advantage for the ADL substrate that lies in using the extra thickness, softness and sponginess that the hydroentanglement technology provides with respect to the rest of the technologies, in order to develop a more efficient ADL substrate and better absorption and distribution properties. A greater dry sensation on the skin is also achieved.

The Spun laid, Wet laid or Air laid technologies are ideal for the development of low thickness and grammage products, objectives that are completely opposite to those presented in the present invention and as may be seen in the comparative table of example 1, wherein for example, during the development of the filtering substrate, it has been seen that for a similar composition type, the thickness and DHC results are considerably lower than when the bonding of fibres is carried out by means of Thermal/Chemical Bonding in comparison with a hydroentanglement or Spunlace.

As such, it may be seen how not only the selection of the material that constitute the layers is essential but the process carried out is also fundamental.

An object of the present invention is a non-woven fabric, used in an air filtering substrate and in an ADL substrate, with a three-dimensional multi-layer structure, made up of 4 overlapping layers of different fibres (solid or hollow), manufactured continuously, progressively and without the use of adhesives between the different layers of fibres. The thickness of the product is from 0.6 to 3 mm and in the case of the filtering substrate, it is preferably from 1.5 to 3 mm (in the ADL substrate preferably from 1.3 to 2.5 mm); moreover, in the case of the filtering substrate, fire retardant substances and PP (polypropylene) fibres to improve the static charge properties thereof may additionally be incorporated; and in the case of the ADL substrate, the upper layers additionally comprising hydrophilic substances and the lowers layers in contact with the absorbent core comprising hydrophobic substances.

The present invention relates, in one aspect, to a non-woven fabric with a three-dimensional multi-layer structure comprising 4 layers arranged one above another of solid, hollow, natural or synthetic fibres wherein there is an arrangement of layers such that the first and second layers are those that are in contact with a flow to be treated, whether filtered or captured and distributed, and comprise fibres of a fineness ranging from 6 to 20 dtex and lengths ranging from 32 to 80 mm, and the remaining layers comprise fibres of a fineness ranging from 0.6 to 10 dtex and lengths ranging from 12 to 64 mm. In the filtering substrate, the flow to be filtered is air and in the ADL substrate it may be, for example, urine.

This particular arrangement of the multi-layer NWF of the present invention, achieves a funnel-type "gradient" effect in the filtering substrate where the largest particles are retained in a first level (first and second layers), without clogging the filtering surface and leaving a passage for the medium and smaller sized particles, which are in turn gradually retained in different levels (subsequent lower layers), but still enabling the passage of air flow with microparticles not affected by the filtering, thus ensuring an efficiency greater than 99% and a DHC higher than 15 g/200 cm².

Preferably, the different layers are similarly formed by fibres of materials selected from the group formed by:
- artificial materials: viscose, glass, silicone, acetate, etc.,
- natural materials: wool, cotton, coconut, sisal, cashmere, asbestos, metal (nickel-chrome, caesium-chrome), ceramics, etc., or
- synthetic materials: polyester, polypropylene, polyamide (Nylon), polyacrylonitrile (acrylic), polyethylene, polycarbonate, etc.

With regards to the ADL substrate, it uses the gradient fibre structure of the filter and the materials selected for the fibres are the same, except in the fact that viscose is not considered since it has a negative effect on the rewet and the dry sensation against the skin. In addition, TWO-COMPONENT fibres may be incorporated in order to provide the fabric with greater resistance; these two components may be of the PET/coPET, PET/PP or PP/PE type.

### EXEMPLARY EMBODIMENTS

The following specific examples that are provided herein serve to illustrate the nature of the present invention. These examples are included for illustrative purposes only, and should not be interpreted as limiting the invention claimed herein.

### EXAMPLE 1A. Comparative study on the thickness and DHC in a fibre bonding method by means of thermal/chemical bonding vs. the method object of the present invention (Souniace).

To carry out the following study, it was checked that the Spunlaid, Wetlaid or Airlaid technologies are ideal for the development of low thickness and grammage products, objectives that are completely opposite to that presented in the present invention.

As may be seen in the following comparative table, during the development we have seen that for a similar composition type, the thickness and DHC results are considerably lower when the bonding of fibres is carried out by means of Thermal/Chemical Bonding in comparison with a hydroentanglement or Spunlace.

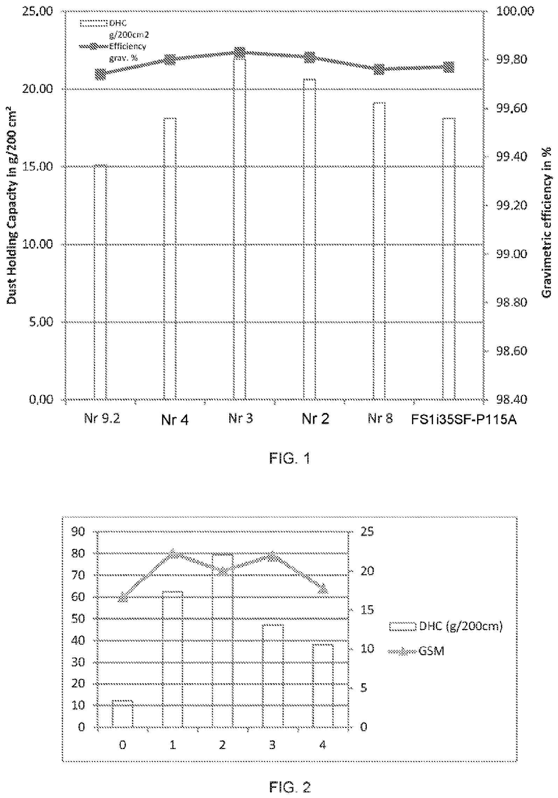

The present table shows the comparison between one of the samples obtained by the method object of the present invention (sample coded as n²3) and a sample obtained by means of the Thermal/Chemical Bonding process (sample coded as n²9.2). The data represented in the following table correlates with figure 1.

| **Technical parameters** | **Sample n**- **3** | **Sample n**- **9**.**2** |
|---|---|---|
| GSM | 72 | 88 |
| Thickness | 1.71 | 1.38 |
| DHC (g/200cm) | 22 | 15 |
| Efficiency (%) | 99.8 | 99.3 |

In this table it may be seen how the filtration efficiency of sample 3 (99.8%) is higher than the result obtained with sample 9.2 (99.3%). Even with lower grammage, the fabric by means of the method of the present invention has greater thickness and DHC than the Thermal Bonding fabric, therefore, with the same material with this multi-layer structure prepare by means of the method of the present invention, a filter with maximum retention and, in turn, minimal clogging, is achieved.

### EXAMPLE 1B. Comparative study on the thickness, grammage and rewet in a fibre bonding method by means of thermal/air bonding vs. the method object of the present invention (Souniace).

To carry out the following study, it was checked that the Spunlaid, Wetlaid or Airlaid technologies are ideal for the development of low thickness and grammage products, objectives that are completely opposite to that presented in the present invention.

As may be seen in the following comparative table, during the development we have seen that for a similar composition type, the thickness and grammage results are considerably lower when the bonding of fibres is carried out by means of Thermal/Through Air Bonding in comparison with a hydroentanglement or Spunlace.

| **TYPE** | **1** | **2** | **3** |
|---|---|---|---|
| Technology | Carded/airlaid thermal bonding | Carded through air bonding | Carded spunlace |
| Weight (GSM) | 45 - 60 | 40-55 | 60 |
| Thickness (mm) | 0.6-1.15 | 0.65-1.15 | >1.3 |

It is especially relevant that the NWF of the present invention is manufactured using this technology, as it provides added sponginess and thickness which facilitates quick capture and diffusion of the urine since it works as an ADL in a nappy, incontinence product, or feminine hygiene product.

In the same way, internal rewet tests carried out during development have clearly shown the differences between using an ADL made exclusively out of spunlace as opposed to all the rest.

| **TYPE** | **1** | **2** | **3** |
|---|---|---|---|
| Technology | Carded/airlaid thermal bonding | Carded through air bonding | Carded spunlace |
| Rewet after 1^{st} urination (g) | 0.16 - 0.23 | 0.15 - 0.23 | <0.1 |
| Rewet after *2nd* urination (g) | 0.23 - 0.28 | 1.7 - 13.8 | <0.23 |

### EXAMPLE 2A: Calculating the optimum parameters for the filtering substrate

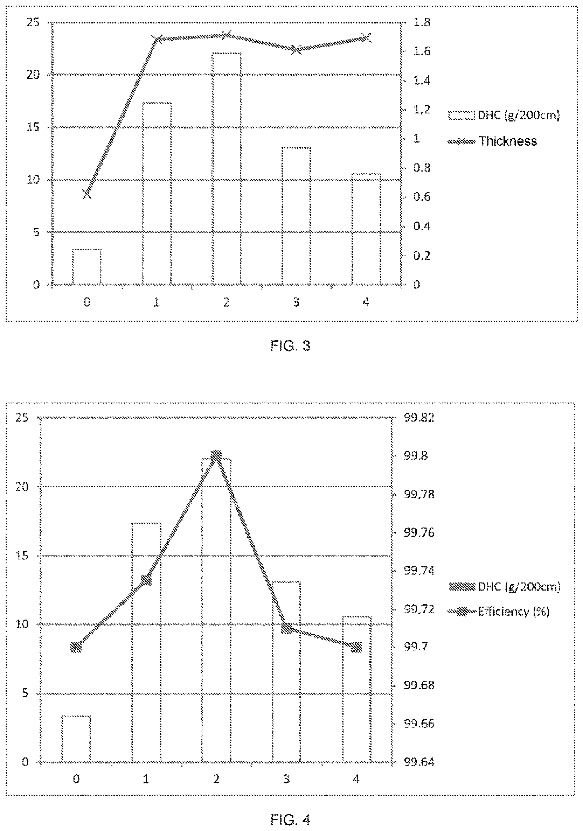

Through the present example the researchers behind the present invention reached the conclusion that the essential technical parameters of the filtering substrate must be as follows:
- The case of the filtering substrate developed has an efficiency higher than 99% and a DHC higher than 15 g/200 cm². Results obtained and validated in the successive tests carried out (see figure 2).
- The GSM must be comprised between 60 and 120 g/m² (see figure 2). Below 60 g/m² the DHC falls below 15 g/200 cm². We have set the upper limit as the line capacity.
- The thickness of the substrate must be comprised between 1 and 3 mm, preferably between 1.5 and 3 mm. Below 1 mm the DHC falls below 15 g/200 cm². We have set the upper limit as the line capacity (see figure 3).

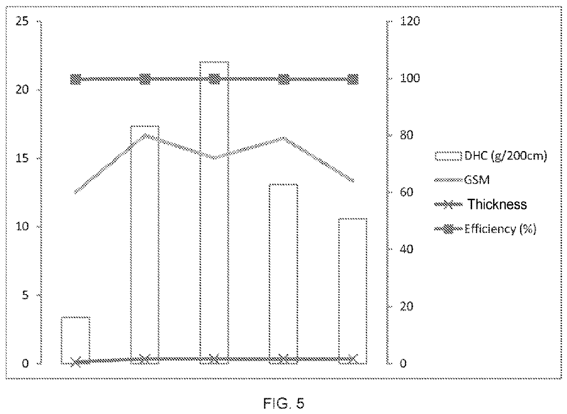

All of these parameters regarding filtration efficiency are represented in figure 5.

### EXAMPLE 2B: Calculating the optimum parameters for the ADL substrate

Through the present example the researchers behind the present invention reached the conclusion that the essential technical parameters of the ADL substrate must be as follows:
- The developed ADL substrate has a rewet of <0.15 g and an STT of <4", according to the results obtained and validated in the successive tests carried out.
- The GSM must be comprised between 30 and 90 GSM. Below 30 GSM, the rewet is excessive (>0.15 g).
- The thickness of the substrate must be comprised between 0.6 and 3 mm, preferably between 1.3 and 2.5 mm. Below 0.6 mm, the rewet is excessive (>0.15 g).
- The dry-bursting and MD (machine direction) tensions must be comprised between 10 and 70 N/5 cm, preferably between 30 and 50 N/5 cm. Below 10 N/5 cm the fabric cannot be processed, and above 70 N/5 cm there is a negative impact on thickness.
- The dry-bursting and CD (cross direction) tensions must be comprised between 2 and 20 N/5 cm, preferably between 5 and 15 N/5 cm. Below 2 N/5 cm the fabric cannot be processed, and above 20 N/5 cm there is a negative impact on thickness.

The parameters employed have therefore been justified experimentally.

### EXAMPLE 3: Manufacturing a non-woven fabric for a filtering substrate and an ADL substrate

In the present example a non-woven fabric has been designed for a filtering substrate and an ADL substrate in accordance with the object of the invention. The first and second layers, both of which are intended to retain the largest particles or capture liquid, are completely formed by natural or synthetic fibres (of the Viscose, PET, PP type or the like; the ADL substrate cannot be made of Viscose) of a fineness ranging from 6 to 20 dtex and lengths ranging from 32 to 80 mm.

In our process, fibres in this fineness range with lengths smaller than 32 mm do not hydroentangle well when passing through the jet. Lengths greater than 80 mm cannot be processed with assurances either in our carding. During development we have observed that by using fibres with finenesses smaller than 6 dtex in this first and second layer, we lose thickness, and therefore DHC in the filtering substrate and rewet in the ADL substrate.

As shown in the table below, at fineness greater than 20 dtex, the space of the weft created between fibres is excessive and the efficiency and DHC fall (see figure 4).

| | **Identification of the samples** | **0** | **1** | **2** | **3** | **4** |
|---|---|---|---|---|---|---|
| Technical parameters | Fineness fibres 1 st and 2nd layer | 1.7 dtex | 3.3-6 dtex | 6-20 dtex | 6 - 20 dtex | 6 - 20 dtex |
| | Fineness fibres in subsequent layers | 1.7 dtex | 1.7-7 dtex | 0.6-7 dtex | 0.6 - 10 dtex | 0.6 - 10 dtex |
| | Batch | Standard split | I0364-5 | I1023-3 | J0561-5 | J1370-2 |
| | Thickness (mm) | 0.62 | 1.68 | 1.71 | 1.61 | 1.69 |
| | DHC (g/200 cm2) | 3.35 | 17.31 | 22 | 13.04 | 10.56 |
| | Efficiency (%) | 99.7 | 99.7 | 99.8 | 99.7 | 99.7 |
| | Rewet (g) | 0.27 | 0.09 | 0.03 | 0.11 | 0.13 |

The third and subsequent layers, which are intended, in the filtering substrate, to retain the largest particles, and, in the ADL substrate, for the final distribution of the urine to the absorbent core, are also formed by natural or synthetic fibres (of the Viscose, PET, PP type or the like; the ADL substrate cannot be made of Viscose) of a fineness ranging from 0.6 to 7 dtex and lengths ranging from 12 to 64 mm. In our process, fibres with a fineness lower than 0.6 dtex or with lengths smaller than 12 mm or greater than 64 mm cannot be processed with assurances when carded. We have also checked that at fineness greater than 7 dtex in the secondary layers, the space of the weft created between fibres is excessive and the efficiency and DHC fall in the filtering substrate and the anti-rewet barrier effect falls in the ADL substrate.

An object of the present invention is a method for producing a non-woven fabric for an air filtering substrate or an ADL substrate with a three-dimensional multi-layer structure, without the use of adhesives or the application of heat between the different layers of fibre, comprising the following stages:
a) selecting component materials of the fibres selected from the group formed by solid, hollow, natural, artificial or synthetic fibres,
b) screening the fibres in stage a) for the purpose of obtaining a distribution of fibres having different dimensions, as well as avoiding any impurities that may be present,
c) carding the fibres, obtaining at least 4 veils or layers,
d) hydroentangling in a three-dimensional manner, thereby joining or bonding the layers obtained in stage c), obtaining a primary substrate,
e) drying and winding to obtain the definitive substrate with a thickness of between 0.6 and 3 mm.

Each carding provides 2 to 3 veils (which we call layers) depending on the number of combers it has. In general, the number of combers varies between 1 and 3, preferably between 2 and 3, such that each carding provides between 1 and 3 layers or veils; preferably between 2 and 3 veils or layers. The jet hydroentanglement process serves to join/bond together all of the veils or layers, but since each veil is made up of fibres of different dimensions, the bonding in each layer happens differently (gradient effect - more space with thick fibres than with fine fibres).

An object of the present invention is a non-woven fabric for an air filtering substrate and in an ADL substrate with a three-dimensional multi-layer structure, made up of at least 4 overlapping layers of different fibres (solid or hollow), manufactured continuously, progressively and without the use of adhesives between the different layers of fibres.

In the case of the filtering substrate, which comprises the foregoing non-woven fabric, the thickness of the product is from 1 to 3 mm, and fire retardant substances and PP (polypropylene) fibres may additionally be incorporated to improve the static charge properties thereof.

Fire-combustion-retardant substances may be incorporated in two ways: via fibres (the polymer of the fibres themselves has been developed to incorporate this additive) or via post-treatment, i.e. adding it in a stage after stage e) of the method that is an object of the present invention.

The present invention therefore relates to a non-woven fabric with a three-dimensional multi-layer structure comprising at least 4 layers, arranged securely one above another, of solid, hollow, natural or synthetic fibres, wherein there is an arrangement of layers such that the first and second layers are those that are in contact with a fluid to be treated, and comprise fibres of a fineness in a range of 6 to 20 dtex and lengths in a range of 32 to 80 mm, and in that the remaining layers comprise fibres of a fineness in a range of 0.6 to 10 dtex and lengths in a range of 12 to 64 mm.

In the air filtering substrate, the first two upper layers may be in contact with the air flow to be filtered.

Preferably, the different layers of the non-woven fabric comprised in the air filtering substrate are similarly formed by fibres of materials selected from the group formed by:
- artificial materials: viscose, glass, silicone, acetate, etc.
- natural materials: wool, cotton, coconut, sisal, cashmere, asbestos, metal (nickel-chrome, caesium-chrome), ceramics, etc., or
- synthetic materials: polyester, polypropylene, polyamide (Nylon), polyacrylonitrile (acrylic), polyethylene, polycarbonate, etc.

In accordance with another aspect, the non-woven air filtering substrate of the present invention has an efficiency greater than 99% with a DHC higher than 15 g/200 cm² and a GSM comprised between 60 and 120 g/m².

In accordance with another aspect, the non-woven air filtering substrate of the present invention additionally comprises fire retardant substances and polypropylene fibres to improve the static charge properties thereof.

In accordance with another aspect, the non-woven air filtering substrate of the present invention has a thickness of between 1 mm and 3 mm. Preferably, between 1.5 and 3 mm.

Another object of the present invention is an acquisition and distribution of liquids (ADL) substrate comprising the non-woven fabric described above, wherein the first two upper layers may be in contact with a bodily fluid to be captured, additionally comprising at least one hydrophilic substance, which preferably may be biodegradable surfactants. These biodegradable surfactants are one among fatty acids and esters or a combination thereof.

In the acquisition and distribution of liquids substrate, the different layers are formed by fibres of materials selected from the group formed by artificial materials of the glass, silicone or acetate type, by natural materials of the wool, cotton, coconut, sisal, cashmere, asbestos, metal (nickel-chrome, caesium-chrome) or ceramic type, or synthetic materials of the polyester, polypropylene, polyamide (Nylon), polyacrylonitrile (acrylic), polyethylene or polycarbonate type.

In accordance with one characteristic of the ADL substrate, the layers of fibres of synthetic materials may has two components, of the polyester - polyester or polyester - polypropylene type.

At least the two lower layers may additionally comprise a hydrophobic substance such as biodegradable surfactants. In the same way as the upper layers, the biodegradable surfactants are one among fatty acids and esters or a combination thereof.

In accordance with another aspect, the ADL substrate has a GSM comprised between 30 and 90 g/m² and a thickness between 0.6 mm and 3 mm, preferably between 1.5 mm and 2.5 mm.

The present invention also relates to a method for producing a non-woven fabric with a three-dimensional multi-layer structure that comprises the following stages:
a) selecting component materials of the fibres selected from the group formed by solid, hollow, natural, artificial or synthetic fibres,
b) screening the fibres in stage a) for the purpose of obtaining a homogeneous distribution of fibres having different dimensions and thicknesses, as well as avoiding any impurities that may be present,
c) carding the fibres, obtaining at least 4 veils or layers,
d) hydroentangling in a three-dimensional manner, thereby joining or bonding the layers obtained in stage c), obtaining a primary substrate,
e) drying and winding to obtain the definitive substrate with a thickness of between 0.6 and 3 mm.

In accordance with another aspect, the method yields a non-woven fabric wherein the first two upper layers that are in contact with the flow to be treated comprise fibres of a fineness ranging from 6 to 20 dtex and lengths ranging from 32 to 80 mm and in that the remaining layers comprise fibres of a fineness ranging from 0.6 to 10 dtex and lengths ranging from 12 to 64 mm.

In accordance with another aspect, in the non-woven fabric for the filtering substrate, the thickness in stage e) is between 1 mm and 3 mm and fire-combustion-retardant substances and/or polypropylene fibres to improve the static charge properties thereof are added, either by means of a stage after the drying and winding stage of the definitive substrate or stage f), or through their incorporation into the initial fibres in stage a).

In the method for producing the non-woven fabric for an acquisition and distribution of liquids substrate, hydrophilic substances are added in at least the two upper layers after stage e) and the two lower layers comprise fibres of a fineness in a range of 0.6 to 7 dtex. Moreover, hydrophobic substances are added in at least the two lower layers.

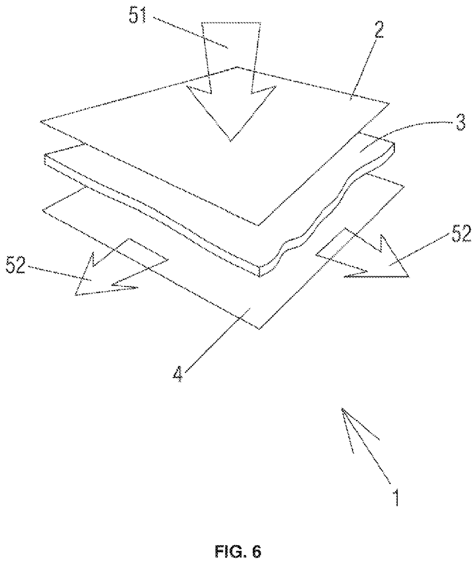

Another object of the invention is a sanitary product 1 comprising an acquisition and distribution of liquids substrate 3 as described above, arranged between a cover 2 and an absorbent core 4, whose structure is illustrated in figure 6. The inner cover 2 or lining that touches a baby's skin is usually made of polypropylene; after this is where the present ADL substrate 3 is situated, and lastly the absorbent core 4 of the diaper contains wood pulp and super-absorbent polymers, generally sodium polyacrylate, which can absorb up to 30 times its weight in urine.

As mentioned previously, the main function of the ADL substrate 3 consists of facilitating urine capture and accelerating its distribution toward the absorbent core 4.

In figure 6 one may observe how a schematic urine flow 51 passes through the cover 2 and immediately afterwards passes into the ADL substrate 3 that comprises the present non-woven fabric. The first two upper layers (not shown) in turn comprise a hydrophilic substance that accelerates the passage of the urine through the gaps between the fibres. As mentioned previously, the two upper layers have wider gaps between the fibres, letting in a greater amount of urine 51, which may be concentrated in a very specific area. Next, the urine descends down to the two lower layers, which have finer gaps, but, thanks to the gradient or funnel effect, the incoming urine flow 51 that enters in a more confined manner in the larger gaps in the upper layers, is quickly distributed by the finer gaps in the lower layers, and from there the outgoing urine flows 52 end up in the absorbent core 4.

According to another aspect, the present invention relates to the use of the filtering substrate to prepare filters for industrial processes where high-efficiency air purification is needed, of the paper, chemical, textile, pharmaceutical or automotive industry type.

## Claims

1. A non-woven fabric with a three-dimensional multi-layer fibre-based structure, comprising at least 4 layers, arranged one above another, arranged securely, **characterised in that** the first two upper layers that are in contact with a flow to be treated comprise fibres of a fineness in a range of 6 to 20 dtex and lengths in a range of 32 to 80 mm, and **in that** the remaining layers comprise fibres of a fineness in a range of 0.6 to 10 dtex and lengths in a range of 12 to 64 mm, wherein the layers are arranged securely without adhesives between them.

2. A non-woven air filtering substrate comprising the non-woven fabric of claim 1, wherein the first two upper layers may be in contact with the air flow to be filtered.

3. The non-woven air filtering substrate according to claim 2, **characterised in that** the different layers are formed by fibres of materials selected from the group formed by artificial materials of the viscose, glass, silicone or acetate type, by natural materials of the wool, cotton, coconut, sisal, cashmere, asbestos, metal (nickel-chrome, caesium-chrome) or ceramic type, or synthetic materials of the polyester, polypropylene, polyamide (Nylon), polyacrylonitrile (acrylic), polyethylene or polycarbonate type.

4. The non-woven air filtering substrate according to claim 2, **characterised in that** it has an efficiency greater than 99% with a DHC higher than 15 g/200 cm² and a GSM comprised between 60 and 120 g/m².

5. The non-woven air filtering substrate according to any of claims 2 to 3, **characterised in that** it additionally comprises fire-combustion-retardant substances and polypropylene fibres to improve the static charge properties thereof.

6. The non-woven air filtering substrate according to claim 2, **characterised in that** it has a thickness between 1 mm and 3 mm.

7. The non-woven air filtering substrate according to claim 2 or 6, **characterised in that** it has a thickness between 1.5 mm and 3 mm.

8. An acquisition and distribution of liquids substrate comprising the non-woven fabric of claim 1, wherein the first two upper layers may be in contact with a bodily fluid to be captured, additionally comprising at least one hydrophilic substance.

9. The acquisition and distribution of liquids substrate according to claim 8, **characterised in that** the different layers are formed by fibres of materials selected from the group formed by artificial materials of the glass, silicone or acetate type, by natural materials of the wool, cotton, coconut, sisal, cashmere, asbestos, metal (nickel-chrome, caesium-chrome) or ceramic type, or synthetic materials of the polyester, polypropylene, polyamide (Nylon), polyacrylonitrile (acrylic), polyethylene or polycarbonate type.

10. The acquisition and distribution of liquids substrate according to claim 9, **characterised in that** the fibres of synthetic materials have two components of the PET/coPET, PET/PP or PP/PE type.

11. The acquisition and distribution of liquids substrate according to claim 8, **characterised in that** the hydrophilic substances are biodegradable surfactants.

12. The acquisition and distribution of liquids substrate according to claim 11, **characterised in that** the biodegradable surfactants are one from among fatty acids and esters or a combination thereof.

13. The acquisition and distribution of liquids substrate according to claim 8, **characterised in that** at least the two lower layers additionally comprise a hydrophobic substance.

14. The acquisition and distribution of liquids substrate according to claim 13, **characterised in that** the hydrophobic substances are biodegradable surfactants.

15. The acquisition and distribution of liquids substrate according to claim 14, **characterised in that** the biodegradable surfactants are one from among fatty acids and esters or a combination thereof.

16. The acquisition and distribution of liquids substrate according to any one of claims 8-15, **characterised in that** it has a GSM comprised between 30 and 90 g/m².

17. The acquisition and distribution of liquids substrate according to any one of claims 8-16, **characterised in that** it has a thickness of between 0.6 mm and 3 mm.

18. The acquisition and distribution of liquids substrate according to any one of claims 8-16, **characterised in that** it has a thickness of between 1.3 mm and 2.5 mm.

19. A sanitary product (1) comprising an acquisition and distribution of liquids substrate (3) according to any one of claims 8-18, arranged between a cover (2) and an absorbent core (4).

20. A method for producing a non-woven fabric with a three-dimensional multi-layer structure of claim 1, **characterised in that** it comprises the following stages:
a) selecting component materials of the fibres selected from the group formed by solid, hollow, natural, artificial or synthetic fibres,
b) screening the fibres in stage a) for the purpose of obtaining a homogeneous distribution of fibres having different dimensions and thicknesses, as well as avoiding any impurities that may be present,
c) carding the fibres, obtaining at least 4 veils or layers,
d) hydroentangling in a three-dimensional manner, thereby joining or bonding the layers obtained in stage c), obtaining a primary substrate,
e) drying and winding to obtain the definitive substrate with a thickness of between 0.6 and 3 mm.

21. The method for producing the non-woven fabric according to claim 20, **characterised in that** a definitive non-woven fabric is obtained wherein the first and second upper layers that are in contact with a flow to be treated comprise fibres of a fineness ranging from 6 to 20 dtex and lengths ranging from 32 to 80 mm and **in that** the remaining layers comprise fibres of a fineness ranging from 0.6 to 10 dtex and lengths ranging from 12 to 64 mm.

22. The method for producing the non-woven fabric according to any one of claims 20 to 21 for an air filtering substrate according to claim 5, **characterised in that** in stage e), the thickness is between 1 and 3 mm and fire-combustion-retardant substances and/or polypropylene fibres to improve the static charge properties thereof are added by means of a stage after the drying and winding stage of the definitive substrate or stage f).

23. The method for producing the non-woven fabric according to claim 22, **characterised in that** fire-combustion-retardant substances and/or polypropylene fibres to improve the static charge properties thereof are added through their incorporation into the initial fibres in stage a).

24. The method for producing the non-woven fabric according to any one of claims 20 to 21 for an acquisition and distribution of liquids substrate according to any one of claims 8-18, **characterised in that** hydrophilic substances are added in at least two upper layers and the two lower layers comprise fibres of a fineness in a range of 0.6 to 7 dtex.

25. The method for producing the non-woven fabric according to any of claims 20 to 21 and 24 for an acquisition and distribution of liquids substrate according to any one of claims 8-18, **characterised in that** hydrophobic substances are added in at least the two lower layers.

26. A use of the filtering substrate of claims 2 to 7 to prepare filters in industrial processes where high-efficiency air purification is needed, of the paper, chemical, textile, pharmaceutical or automotive industry type.
